# FASCICULE DE BREVET EUROPEEN

(11) **EP 1 314 727 B1**
(45) Date de publication et mention de la délivrance du brevet: **08.09.2010**
(21) Numéro de dépôt: 02292871.7
(22) Date de dépôt: 19.11.2002
(51) Int. Cl.: C07D 221/12, C07F 5/02

(54) **Synthèse directe de sels quaternaires de phenanthridinium**
Direktsynthese von quaternären Phenanthridiniumsalzen
Direct synthesis of quaternary phenanthridinium salts

(30) Priorité: 23.11.2001 FR 0115200
(43) Date de publication de la demande: 28.05.2003
(73) Titulaire: MERIAL, 69007 Lyon (FR)
(72) Inventeur: Capelle, Nicolas, 30000 Nimes (FR); Gallo, Roger, 13320 Bouc Bel Air (FR)
(74) Mandataire: Nargolwalla, Cyra

(56) Documents cités:
- WO-A-95/21227
- J.J.S. LAMBA ET AL: "Imine-bridged planar poly(p-phenylene) derivatives for maximization of extended pi-conjugation. The common intermediate approach" JOURNAL OF THE AMERICAN CHEMICAL SOCIETY., vol. 116, no. 26, 1994, pages 11723-11736, XP002202565 DC US
- M.A. SIDDIQUI ET AL: "The directed metalation connection to aryl-aryl cross coupling. Regiospecific synthesis of phenanthridines, phenanthridinones and the biphenyl alkaloid ismine" TETRAHEDRON LETTERS, vol. 29, no. 43, 1988, pages 5463-5466, XP002202566 OXFORD GB
- T.I. WATKINS: "Trypanocides of the phenanthridine series. The effect of changing the quaternary grouping in dimidium bromide" JOURNAL OF THE CHEMICAL SOCIETY., 1952, pages 3059-3064, XP002202567 LETCHWORTH GB
- L. GHOSEZ ET AL: "Studies of palladium-catalyzed coupling reactions for preparation of hindered 3-arylpyrroles relevant to (-)-rhazinilam and its analogues" CANADIAN JOURNAL OF CHEMISTRY., vol. 79, no. 11, 2001, pages 1827-1839, XP002232896 NATIONAL RESEARCH COUNCIL. OTTAWA., CA ISSN: 0008-4042
- CHEMICAL ABSTRACTS, vol. 46, no. 22, 25 novembre 1952 (1952-11-25) Columbus, Ohio, US; abstract no. 10998g, Z. HOLZBECHER: "Reactions of phenylboric acid and its nitro derivatives with metal salts" XP002232897 & CHEM. LISTY, vol. 46, 1952, pages 17-19,

## Description

L'invention a pour objet principal une nouvelle voie d'accès à des sels quaternaires de phénanthridinium et vise également un procédé de préparation d'intermédiaires réactionnels de cette voie de synthèse.

Les sels quaternaires de phénanthridinium sont des intermédiaires de synthèse particulièrement recherchés dans la mesure où d'une part ils figurent des précurseurs de médicaments utiles en santé animale comme trypanosomicides et d'autre part sont eux-mêmes actifs comme dérivés pharmaceutiques contre la maladie du sommeil. Ils sont également utilisés comme médicaments intercalants de l'ADN et/ou agents anti-tumoraux pour le traitement des maladies parasitaires.

Les phénanthridines sont généralement synthétisées par une réaction de couplage de Suzuki entre un acide boronique contenant un groupement azote protège en position ortho et un dérivé halogénoaryle contenant un groupement carbonyle en position ortho, suivie d'une réaction de déprotection de l'azote et d'une cyclisation par déshydratation. Lamba et Tour (J. Am. Chem. Soc. 1994, 116, 11723) décrivent une réaction de Suzuki, impliquant une aniline protégée par un groupement BOC et une cétone méthylée, dans laquelle un traitement acide ultérieur provoque la perte du groupement BOC et une cyclisation simultanée conduisant au produit désiré. Siddiqui et Snieckus (Tet. Lett. 1988, 29(43), 5463) décrivent des procédés similaires utilisant des aldéhydes aromatiques et fournissent des exemptes dans lesquels un chauffage prolongé conduit à la libération du groupement BOC et à la formation de phénanthridine due de la réactivité élevée du groupement aldéhyde vis-à-vis de la cyclodéshydratation. Watskins (J. Chem. Soc. 1952, 3059) a démontré une quaternisation directe de dérivés de phénanthridine en utilisant des dérivés de p-toluèncsulfonate d'alkyle, tel que le tosylatc d'éthyle, ou des alkyles halogénés. Cependant cette réaction résulte très souvent en une décomposition autocatalytique du produit désiré ou en une déshydratation incomplète produisant une phénanthridine hydratée. Ghosez (Can. J. Chem. 2001, 79, 1827) décrit le couplage de pyrroles substitués d'un halogène et d'un aldéhyde avec des acides boroniques d'aniline suivi d'une déshydratation donnant des dérivés pyrrolo-quinolines.

Les sels quaternaires de phénanthridinium peuvent être représentés comme suit :

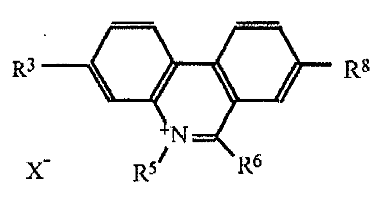

D'une manière générale, ces sels quaternaires de phénanthridinium sont obtenus par quaternisation de la phénanthridine correspondante. Toutefois, cette voie d'accès conventionnelle ne donne pas totalement satisfaction notamment pour les raisons suivantes :

Tout d'abord, les phénanthridines sont des hétérocycles peu réactifs dans la réaction de quaternisation car, d'une part, leur basicité est faible (pKa = 4,47) et, d'autre part, l'encombrement stérique de tout substituant adjacent à l'atome d'azote (R⁶) diminue la réactivité nucléophile de l'hétérocycle. Pour surmonter ce défaut de réactivité naturel, il s'avère nécessaire d'effectuer les quaternisations de dérivés de la phénanthridine à température élevée voire sous pression si nécessaire. Ainsi, dans le cas de la méthylation, la réaction de la phénanthridine avec l'iodure de méthyle (PE-41°C), est réalisée à 110°C. Pour les dérivés alkylés de la phénanthridine, les cinétiques effectuées avec l'iodure de méthyle sont réalisées à 60-95°C en ampoules scellées.

Cette difficulté est par ailleurs accentuée dans le cas de dérivés de la phénanthridine dont la réactivité nucléophile est diminuée (comme pour tous les autres composés azaaromatiques), par la présence de groupements électroattracteurs tels que les groupes nitro. C'est ainsi que la phénanthridine portant un groupement métanitrophényle en R⁶ est quaternisée par le sulfate de méthyle dans le nitrobenzène à 150°C. De même, la phénanthridine portant deux groupes nitro en R³ et R⁸ et un groupement paracyanophényle en R⁶ nécessite une température de 180°C pour être quaternisée par le sulfate de méthyle.

Enfin, cette difficulté à quaterniser les dérivés de la phénanthridine est significativement accrue dans le cas des alkylations visant à introduire d'autres groupements alkylés plus volumineux que le groupement méthyle et, en particulier, le groupement éthyle que représente le groupe R⁵ et qui permet d'atteindre les dérivés homidium et isométamidium.

En effet, si les quaternisations sont ralenties par l'effet réductif (attracteur) de certains substituants (qui diminuent la basicité de l'hétérocycle) et l'effet stérique des substituants R⁶ en ortho (qui empêchent l'approche du réactif d'alkylation), elles le sont également en raison de l'effet stérique du réactif d'alkylation. C'est ainsi que pour introduire le groupement éthyle, les halogénures d'éthyle, chlorure, bromure et iodure d'éthyle, nécessitent de travailler sous pression.

En conséquence, il serait particulièrement avantageux de disposer d'une nouvelle voie d'accès aux sels quaternaires de phénanthridinium permettant d'une part de s'affranchir de cette opération de quaternisation dont la mise en oeuvre s'avère difficile et dangereuse et d'autre part d'obtenir des sels de phénanthridinium difficilement synthétisables aujourd'hui par la voie conventionnelle pour des raisons d'encombrement stérique et/ou de réactivité défavorables. C'est notamment le cas des sels de phénanthridinium, portant des groupements nitro et qui sont des intermédiaires de synthèse de trypanosomicides comme les dérivés homidium, dimidium ou isometamidium.

Un autre objectif de la présente invention est d'éviter l'emploi de produits de départ toxiques.

Plus particulièrement, la présente invention a pour objet principal un procédé utile pour la préparation de sels quaternaires de phénanthridinium de formule générale (I) : 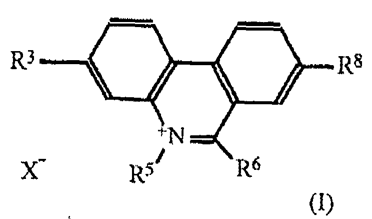 dans laquelle :
- au moins un des groupements R³ et R⁸ représente un groupement nitro (NO₂),
- R⁵ représente un groupement méthyle ou éthyle,
- R⁶ représente un groupement aryle ou hétéroaryle en C₅ à C₁₂, condensé ou non, et pouvant incorporer un ou plusieurs hétéroatomes et,
- X⁻ représente un anion halogénure ou hydrogénosulfate ou la base conjuguée d'un acide minéral
caractérisé en ce qu'il comprend la réaction d'au moins un dérivé halogénoaromatique de formule (II) : 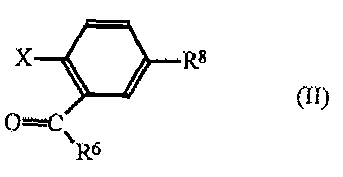 dans laquelle R⁸ et R⁶ sont tels que définis précédemment et X représente un atome d'halogène avec au moins un dérivé phénylboronique de formule (III) : 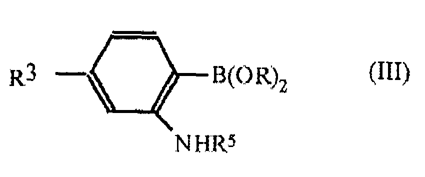 dans laquelle :
- R³ et R⁵ sont tels que définis précédemment et,
- les deux groupements R représentent un atome d'hydrogène ou figurent ensemble un radical cyclique de formule : 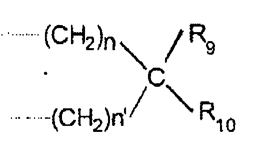
dans laquelle :
- n et n' représentent indépendamment l'un de l'autre un entier compris entre 0 et 4 et R₉ et R₁₀ figurent indépendamment l'un de l'autre un atome d'hydrogène ou un groupement alkyle linéaire ou ramifié en C₁ à C₅,
en milieu basique et en présence d'une quantité efficace d'un catalyseur de palladium et, en ce que l'on récupère ledit produit de formule générale I.

Les inventeurs ont ainsi mis en évidence qu'il était possible d'accéder efficacement aux sels quaternaires de phénanthridinium en reproduisant la réaction dite de SUZUKI sur des substrats de formule générale II. La présence d'un radical alkyle au niveau de l'atome d'azote figurant la fonction amine permet avantageusement de stabiliser cette fonction amine au cours de la réaction de condensation. De manière inattendue, l'élimination de ce groupement n'est pas observée au cours de la réaction de condensation qui en l'occurrence conduit avantageusement à la formation du sel quaternaire de phénanthridinium.

Dans les composés de formule générale (I), l'ensemble des groupements peut être substitué et les chaînes alkyles interrompues par un hétéroatome comme l'azote, le soufre et/ou l'oxygène.

Au moins l'un des groupements R³ et R⁸ et de préférence les deux groupements figurent un groupement nitro.

En ce qui concerne R⁵, il représente un groupement méthyle ou éthyle.

Quant à R⁶ il s'agit de préférence d'un groupement phényle.

Selon un mode de réalisation préférentiel, on met en présence au moins 1 équivalent de composé (II) pour 0,8 à 1,5 et de préférence environ 1,2 équivalent de composé (III).

Une grande variété de catalyseurs à base de Palladium (o) ou de précurseurs de ceux-ci peut être utilisée dans le cadre de la présente invention. Comme catalyseur, convient tout particulièrement Pd(PPh₃)₄.

Toutefois, les catalyseurs de type Pd Cl₂ (PPh₃)₂ et Pd (OAc)₂ + PPh₃ ou autres ligands phosphine s'avèrent également efficaces dans la mesure où ils sont stables à l'air et facilement réduits en complexes actifs Pd (o). A titre représentatif de ces catalyseurs on peut notamment citer les catalyseurs PdCl₂ dppf, PdOAcP(o-butyl)₃, Pd₂(DBA)₃ ou Pd(OAc)₂ avec ajout de ligands tels que PdtBu₃, PCy₃, P(o-tolyl)₃, TPPTs (triphénylphosphine trisulfonate, dppp et dppb (diphénylphosphinopropane ou butane).

Le mélange est chauffé à 80-100°C pendant 8 à 30 heures. Le sel de phénanthridinium est récupéré après acidification avec de l'acide chlorhydrique.

Le catalyseur est mis en oeuvre à raison de 0,1 à 10 % et plus préférentiellement à raison de 5 à 10 % par rapport au composé de formule générale I .

En ce qui concerne la base, elle possède de préférence un pKa de 7 à 17. A titre représentatif des bases convenant à l'invention, on peut plus particulièrement citer les hydroxydes alcalins, les carbonates et hydrogénocarbonates, les alcoolates, les phosphates, ainsi que les bases azotées aliphatiques ou cyclaniques. Conviennent notamment à l'invention, les bases choisies parmi Na₂CO₃, NaHCO₃, K₂CO₃, NaOH, Ba(OH)₂, K₃PO₄, CsF, Cs₂CO₃, Ag₂CO₃, NaMeO, NatBuO, et plus particulièrement, Cs₂CO₃, Et₃N et Bu₃N.

La base est généralement introduite à raison de 2 à 6, et de préférence de 2 à 3 équivalents molaires par rapport au substrat de formule générale (I). Préférentiellement, on utilise deux équivalents molaires de base. Le premier équivalent est utilisé pour augmenter la réactivité de l'acide boronique et le second équivalent à titre de ligand X⁻ sur le complexe ArPdX, issu de l'addition oxydante du dérivé II sur le catalyseur palladium.

Selon une variante particulière de l'invention, on utilise deux équivalents molaires de Cs₂ CO₃.

En ce qui concerne le solvant, il peut s'agir d'un solvant protique ou aprotique. Il est bien entendu choisi de manière à ne pas interférer avec la réaction considérée. Il peut ainsi s'agir d'un solvant aromatique comme le benzène ou le toluène, du diméthylformamide, la N-méthylpyrrolidone, le diméthoxyéthane, un alcool ou un solvant cétonique.

La réaction est réalisée préférentiellement sous atmosphère inerte pour éviter la dégradation du catalyseur au palladium. Toutefois, selon le type de catalyseur considéré, cette atmosphère inerte peut être facultative.

La réaction peut être conduite à une température comprise entre 60 et 130°C. Selon une variante privilégiée de l'invention, le milieu réactionnel est chauffé à une température supérieure à 60°C, de préférence supérieure à 80°C, et plus préférentiellement comprise entre 80°C et 100°C.

Bien entendu, l'ajustement de cette température est fonction du substrat à préparer, du catalyseur et/ou de la base utilisé(s) et relève des compétences de l'homme de l'art.

Le sel de phénanthridinium est récupéré après acidification du milieu réactionnel et selon des techniques conventionnelles.

En ce qui concerne l'ordre d'introduction des réactifs, il n'est pas déterminant. Toutefois, selon une variante préférée de l'invention, on privilégie le mélange des composés de formule générale (II) et (III) dans le solvant considéré. Y sont ensuite ajoutés, la base et le catalyseur retenus.

Selon une variante privilégiée de l'invention, le procédé comprend :
- Le mélange des composés de formule générale (II) et (III) dans un solvant,
- L'ajout au dit mélange de la base et du catalyseur,
- Le chauffage de l'ensemble du milieu réactionnel à une température supérieure à 60°C et,
- La récupération du composé de formule générale (I) par acidification du milieu réactionnel.

Le procédé revendiqué est tout particulièrement utile pour préparer le sel de 3,8-dinitro-5-éthyl-6-phénylphénanthridinium.

La présente invention a également pour objet un procédé de préparation d'intermédiaires utiles pour accéder au composé de formule générale (III).

Les composés de formule générale (III) sont préparés selon le schéma réactionnel suivant : 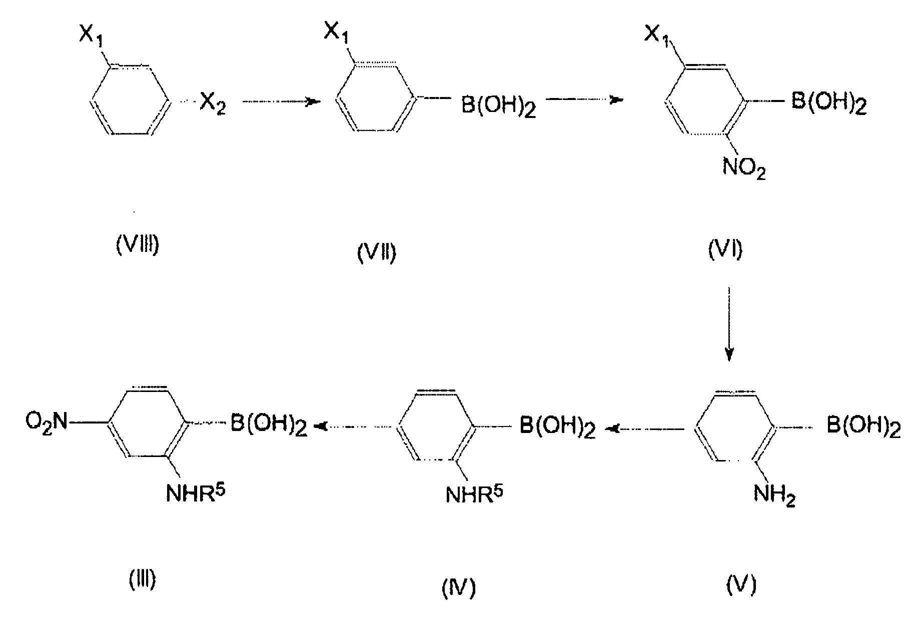 dans lequel X₁ et X₂ représentent indépendamment l'un de l'autre un atome d'halogène ; par exemple, X₁ peut représenter un atome de chlore et X₂ un atome de brome ou X₁ représente un atome de brome et X₂ un atome d'iode.

Les inventeurs ont ainsi mis en évidence qu'il était possible d'accéder régiosélectivement aux composés de formule générale (VI), à partir de composés de formule générale (VII), sous réserve de réaliser la nitration dans des conditions spécifiques.

En l'occurrence, la préparation d'un composé de formule générale (VI) : 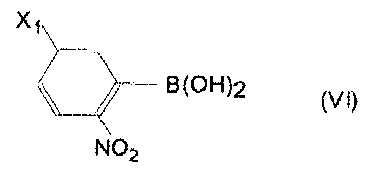 dans laquelle X₁ représente un atome d'halogène, de préférence, un atome de chlore, comprend la nitration d'un composé de formule générale (VII) : 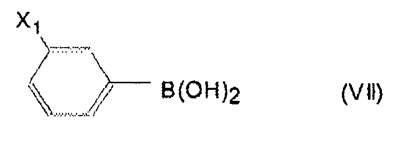 dans de l'anhydride acétique ou un solvant analogue.

Généralement, les nitrations sont conduites avec un mélange HNO₃-H₂SO₄. Compte tenu du caractère méta orientateur du (BOH)₂, et du caractère para et/ou ortho-orientateur de l'atome d'halogène, l'ensemble des positions libres sur le composé sont susceptibles de réagir de manière équivalente vis-à-vis de NO₂⁺ et de conduire à un mélange de 3 composés.

De manière inattendue, l'utilisation du solvant spécifique anhydride acétique, induit une régiosélectivité au niveau de la réaction qui conduit majoritairement au composé de formule générale VI.

En réalisant la nitration d'un mélange HNO₃/Ac₂O, à 0-5°C, on obtient ainsi l'acide 5-chloro-2-nitrophénylboronique (VI) avec une régiosélectivité supérieure à 95%.

La présente invention a également pour objet les composés de formule (VI) 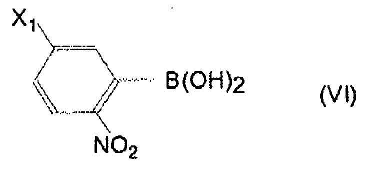 dans laquelle X₁ représente un atome d'halogène.

Elle vise plus particulièrement l'acide 5-chloro-2-nitrophénylboronique.

En ce qui concerne le produit de départ, à savoir le composé (VII), il peut être obtenu au préalable en réalisant un organomagnésien sur son dérivé métabromochlorobenzène de formule VIII, selon une procédure classique.

Ce composé est ensuite hydrodéchloré, selon une technique conventionnelle. Cette réduction peut être réalisée par hydrogénation avec H₂, sous une pression de 7 bars à 40°C dans du méthanol sur Pd/C en présence de triéthylamine. Ces conditions de réaction permettent avantageusement d'effectuer simultanément la réduction de la fonction nitro en fonction amino et l'hydrogénodéchloration.

La monoalkylation au niveau de l'azote du dérivé (V) pour donner l'acide 2-alkylaminophénylboronique (IV) est réalisée par imination réductrice dans le THF à 0-10°C. En ajoutant un aldéhyde RCHO à (V), on obtient l'imine qui est réduite *in situ* par NaHB(OAc)₃ pour conduire à (IV) en limitant à moins de 2% la réaction consécutive de dialkylation de l'amine.

La nitration de (IV) par le mélange acide nitrique / acide sulfurique s'effectue à 0-5°C ; elle conduit à l'acide 2-alkylamino-4-nitrophénylboronique (III) avec un bon rendement.

On peut également envisager de réaliser la nitration finale préalablement à l'alkylation. Toutefois, la transformation dans cet ordre inverse conduit à un moins bon rendement et nécessite une plus grande quantité de réactif pour alkyler l'amine aromatique préalablement nitrée dont la réactivité nucléophile est fortement réduite de ce fait.

Par contre, pour améliorer la transformation de (V) en (III), on peut préparer préalablement l'ester cyclique de (V) par réaction avec le 2,2-diméthyl-1,3-propane diol dans le THF pour donner le 5,5-diméthyl-2(2-aminophényl)-1,3,2 dioxaborinane.

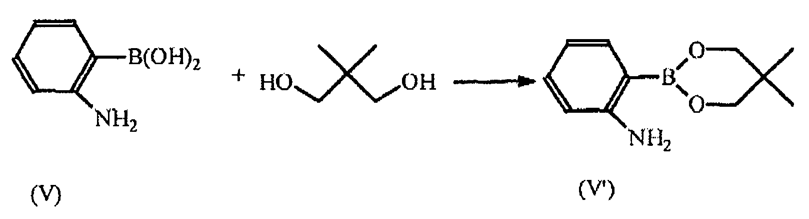

Avec (V') la monoalkylation de l'amine est effectuée plus facilement et avec une proportion moindre de réactif RCHO / NaHB(OAc)₃.

La préparation de II est faite facilement, selon une procédure connue, à partir de l'acide 2-halogéno-5-nitro-benzoïque (IX) par réaction avec SOCl₂ pour former *in situ* le chlorure d'acide auquel on ajoute du benzène et AlCl₃ pour conduire à la 2-bromo-5-nitro-benzophénone (II) avec un rendement de 75%.

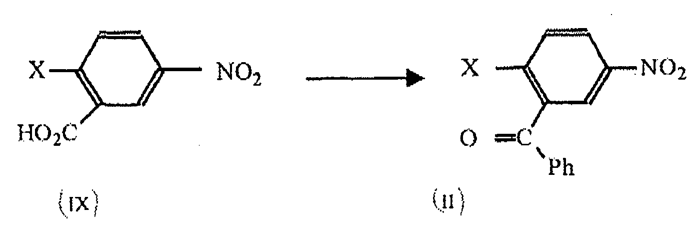

Les exemples suivants illustrent l'invention sans toutefois en limiter la portée.

### EXEMPLE 1 :

### Préparation de l'acide 2-aminophénylboronique (V) par la séquence de réactions (VIII)→(VII)→(VI)→(V).

- 1^{ère} étape - préparation de l'acide métachlorophénylboronique (VII) à partir de (VIII). 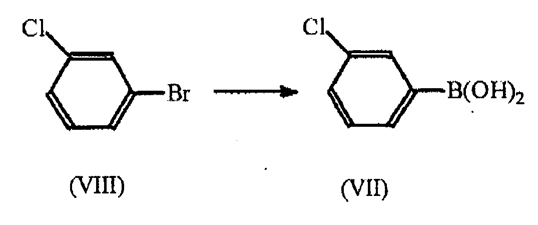 L'organomagnésien de VIII est formé par l'addition de 100 g (0,52 mole) de métabromochlorobenzène dans 250 ml d'éther anhydre sur 12,7 g (0,52 mole) de magnésium recouvert d'éther anhydre et contenant un cristal d'iode. A la fin de l'addition à reflux, le milieu est maintenu 2 h à reflux puis refroidi à température ambiante.
   L'organomagnésien ainsi formé et 57 g (0,53 mole) de triméthylborate sont additionnés en alternance par petites portions sur 250 ml d'éther anhydre, refroidi à -60°C, pendant environ 1 heure. A la fin de l'addition, on laisse revenir le milieu à 0°C. 35 g d'eau sont ajoutés puis un mélange de 300 g d'eau et de 25,6 g d'acide sulfurique concentré sont également ajoutés en maintenant la température inférieure à +15°C. La solution est agitée 2 heures supplémentaires. Les deux phases sont décantées. La phase aqueuse est extraite 3 fois avec 50 ml d'éther. Les phases organiques regroupées sont évaporées. Vers la fin de la distillation de l'éther, 290 g d'eau sont ajoutés goutte à goutte. Le chauffage est poursuivi jusqu'à 97-98°C. Après refroidissement à température ambiante, le produit est récupéré par filtration. Le solide est lavé 2 fois par 150 ml d'éther de pétrole. Après séchage, on récupère 77 g d'une poudre blanche (rendement de 95%) qui correspond à l'acide 3-chlorophénylboronique (VII).
   RMN¹H (DMSO - d6) δ 8,3 (s.2H, OH) ; 7,8 (s, 1H, ArH) ; 7,7 (d, J=6,8 Hz, 1H, ArH) ; 7,3-7,5 (m, 2H, ArH) ; RMN¹³C (DMSO-d6) δ 133,7 (CH) ; 132,9 (C-Cl) ; 132,7 (CH) ; 130,0 (CH) ; 129,7 (CH).
- 2^{ème} étape - préparation de l'acide 5-chloro-2-nitrophénylboronique (VI) à partir de (VII). 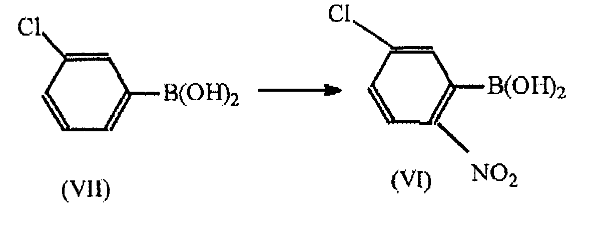 Une suspension de 50g (0,30 mole) d'acide m-chlorophénylboronique dans 160g d'anhydre acétique est refroidie à 0-5°C. Quelques grains de nitrite de sodium sont ajoutés. 30,2g (0,48 mole, 1,5 éq) d'acide nitrique concentré sont ajoutés lentement en maintenant la température du milieu autour de 5°C.
   Il y a successivement précipitation puis homogénéisation du milieu. On laisse le mélange est laissé revenir lentement à température ambiante. L'agitation est poursuivie 2 heures supplémentaires. Il y a une légère exothermie à 25-30°C et reprécipitation du milieu. Le mélange à 20°C est versé sur 400g d'eau. Une solution de 131,4g de soude et 300g d'eau est ajoutée goutte à goutte en maintenant la température autour de 10°C. Le pH est contrôlé au papier pH et ajusté autour de 7 par addition de soude à 30%. Le milieu hétérogène obtenu est extrait par 400 ml puis 2 x 50 ml de MTBE (méthyl tert butyl éther).
   Après évaporation du solvant, on obtient 70g d'un solide jaune (rendement supérieur à 90%).
   RMN¹H(DMSO-d6) δ 8,4 (s, 2H, OH), 8,15 (d, J=8,6 Hz, 1H, ArH), 7,6-7,7 (m, 2H, ArH) ; RMN¹³C(DMSO-d6) δ 148,6 (C-NO₂), 139,6 (C-Cl), 132,0 (CH), 129,1 (CH), 124,9 (CH).
- 3^{ème} étape - préparation de l'acide 2-aminophénylboronique (V) à partir de (VI). 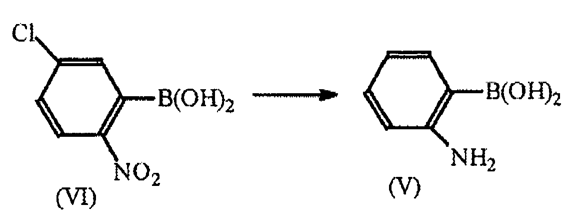 Dans un autoclave, un mélange d'acide 5-chloro-2-nitro-phényl-boronique (10 g ; 0,05 mole), de triéthylamine ( 5,7 g ; 0,055 mole), et de charbon palladié à 5% (1 g) dans 50 ml de méthanol, est placé sous une pression de 7 bar de dihydrogène. L'exothermie des premières 30 min. est contrôlée pour maintenir le milieu à 38-40°C. Le chauffage à 38-40°C est ensuite poursuivi pendant 2 heures. L'évolution de la réaction est contrôlée par CPG par dérivatisation en ester. A la fin de la réaction et après refroidissement à 20°C, le charbon palladié est éliminé par filtration sur célite. Le méthanol est évaporé sous vide, et il est remplacé par de l'acétate d'éthyle (50-60 ml). De l'eau (40 ml) est ajoutée, et les deux phases sont décantées. La phase aqueuse est extraite avec 2 fois 10 ml d'acétate d'éthyle. Le solvant est concentré sous vide, et en fin de concentration de l'éther de pétrole (50 ml) est ajouté. Le produit (2-dihydroxy-boranyl-aniline) est récupéré par filtration pour donner après séchage 2,5 g d'un solide beige (rendement = 37 % pour une pureté > 95%).
   RMN¹H(DMSO-d6/D₂O) δ 7,51 (d, J=7,2Hz, 1H, ArH), 7,1 (pseudo t, Japp = 7,7 Hz, 1H ArH), 6,53-6,32 (m, 2H, ArH) ; RMN¹³C(DMSO-d6/D₂O) δ 154,8 (C), 136,6 (CH), 132,3 (CH) 116,3 (CH), 115,4 (CH).

### EXEMPLE 2 :

### Préparation de l'acide 2-éthylamino-4-nitro-phénylboronique (III) par la séquence de réactions (V) → (IV) → (III).

- 4^{ème} étape - préparation de l'acide 2-éthylaminophénylboronique (IV) à partir de (V). 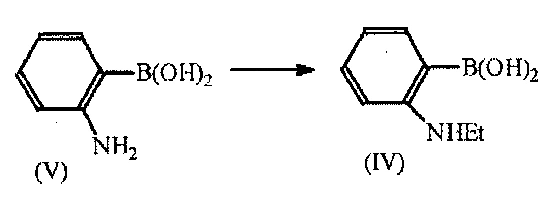 Sur une suspension de 0,55g (10,5 mmoles, 2 éq) de borohydrure de sodium dans 10 ml de THF, 3,5g (53,5 mmoles, 8 éq) d'acide acétique sont ajoutés lentement à une température inférieure à 10°C. 1g (7,3 mmoles) d'acide 2-aminophénylboronique dissous dans 5 ml de THF, est additionné à la suspension obtenue de triacétoxyborohydrure de sodium. 1,61g (36,5 mmoles, 5 éq) d'acétaldéhyde sont ajoutés goutte à goutte à une température inférieure à 10°C. Après 30 minutes d'agitation à 10°C, 5,9 g de bicarbonate de potassium dans 77 ml d'eau sont versés goutte à goutte. Les deux phases sont décantées. La phase aqueuse est extraite avec 2 fois 10 ml d'acétate d'éthyle. Les solvants des phases organiques regroupées sont évaporés sous vide. En fin de concentration, 30 ml d'éther de pétrole sont ajoutés. Après filtration et séchage, 1,4g d'un solide beige est récupéré, soit un rendement supérieur à 90%.
   RMN¹H(DMSO-d6/D₂O/CF₃COOD) δ 8,0 (d,1H,ArH), 7,7 (d,1H, ArH), 7,5-7,6(m, 2H,ArH), 3,6(9,2H,CH₂), 0,95(t, 3H,CH₃) ; RMN¹³C(DMSO-d6/D₂O) δ 156,3 (C-NH₂), 134,9 (CH), 131,7 (CH), 125,4 (CH), 122,6 (CH) ; dérivatisation en ester de 2,2-diméthyl-1,3-propanediol, EI MS m/z (intensité relative) 233 (M⁺, 41), 218(91), 132(100), 105(22), 77(11), 41(22).
- 5^{ème} étape - préparation de l'acide 2-éthylamino-4-nitrophénylboronique (III) à partir de (IV). 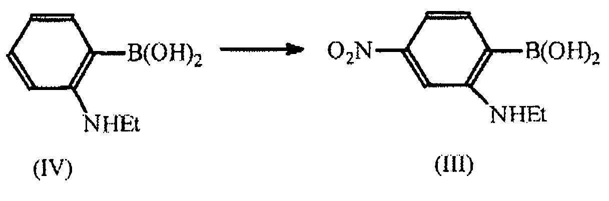 1g (6 mmoles) d'acide 2-N-éthylaminophénylboronique est dissous à froid dans 6g d'acide sulfurique à 98%. Une solution d'acide nitrique 98% (0,42g, 6,6 mmoles, 1,1 éq), et d'acide sulfurique à 98% (1,31g) préalablement mélangés, est additionnée goutte à goutte à 0-5°C.
   L'agitation est poursuivie pendant 2 heures. Le milieu réactionnel est versé goutte à goutte sur 10g d'eau froide à 0-10°C. Le mélange est neutralisé (pH 7) par addition d'environ 10g d'ammoniaque à 32%.
   Le précipité est filtré, lavé par 5 ml d'eau, et séché par distillation azéotropique de l'eau avec du MTBE. Après filtration et séchage, 0,9g de solide marron sont obtenus, soit un rendement de 70%.
   RMN¹³C(DMSO-d6) 158,9(Cl, 129,5, 125,6, 113,9, 108,5, 45,6 (CH₂), 12,2 (CH₃) ; après dérivatisation en ester de 2,2-diméthylpropane-1,3-diol, EI MS m/z (intensité relative) 278 (M⁺, 54), 263(100), 207(51), 177(72), 131(21), 69(35), 41(58).

### EXEMPLE 3 : (variante de l'exemple 2)

### Préparation de l'acide 2-éthylamino-4-nitro-phénylboronique (III) par la séquence de réactions (V) → (V') → (IV') → (III).

- 1^{ère} étape - préparation du 2-(2-aminophényl)-5,5-diméthyl-1,3,2-dioxaborinane (V') à partir de (V). 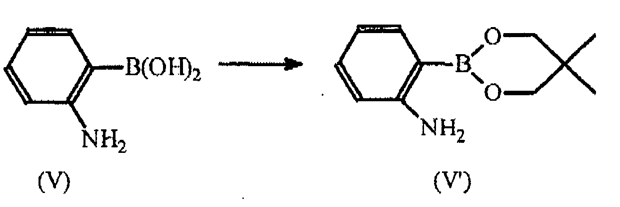 Un mélange d'acide 2-aminophénylboronique (2g, 0,015 mole) et de 2,2-diméthylpropane 1,3-diol (1,57g, 0,015 mole) est dissous dans 10ml de THF. Le solvant est évaporé sous vide, de l'éther de pétrole est ajouté. Après filtration et séchage, 3g de solide beige sont obtenus, soit un rendement supérieur à 95%.
   RMN¹H (DMSO-d6) δ 7,45(dd, J=7,4 Hz, J₂=1,6Hz), 1H, ArH), 7,05 (td, J=7,8Hz, J₂=1,6Hz, 1H, ArH), 6,55 (pseudo d, 1H, ArH), 6,45 (pseudo t, 1H, ArH), 3,7 (s, 4H, CH₂), 0,95 (s, 6H, CH₃) RMN¹³C(DMSO-d6) δ 154,4 (C-NH₂), 135,6(CH), 131,7(CH), 114,9(CH), 114,5(CH), 71,4(CH₂), 31,3(C), 21,3(CH₃). EI MS m/z (intensité relative) 205 (M⁺, 100), 143(14), 119(100), 92(18), 41(26).
- 2^{ème} étape - préparation de 5,5-diméthyl-2(2-éthylaminophényl)-1,3,2-dioxaborinane (IV') à partir de (V'). 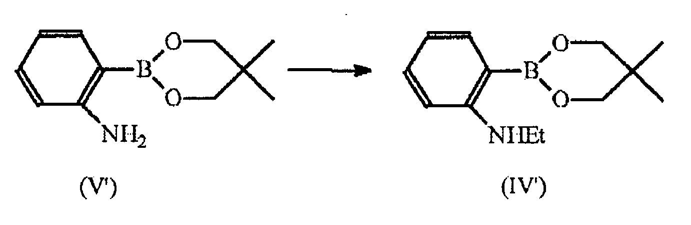 De l'acide acétique (2g, 4,5 éq) est ajouté lentement à 0-10°C à une suspension de borohydrure de sodium (0,42g, 1,5 éq) dans 5 ml de THF. Une solution de 2-amino boronate de 2,2-diméthyl-1,3-propanediol (néopentyl glycol) (1,5g, 7,3 mmoles), dissous dans 5 ml de THF, est ajoutée goutte à goutte. De l'acétaldéhyde (0,48g, 1,5 éq) est additionné lentement à 0-10°C. Le mélange est traité par 3,34g de bicarbonate de potassium dissous dans 17g d'eau. Les deux phases sont décantées. La phase aqueuse est extraite par du MTBE. Les solvants sont évaporés sous vide. Le solide obtenu est repris dans l'éther de pétrole pour donner après filtration et séchage 1,8g d'un solide beige, soit un rendement supérieur à 90%.
- 3^{ème} étape - préparation de l'acide 2-éthylamino-4-nitro-phénylboronique (III) à partir de (IV'). 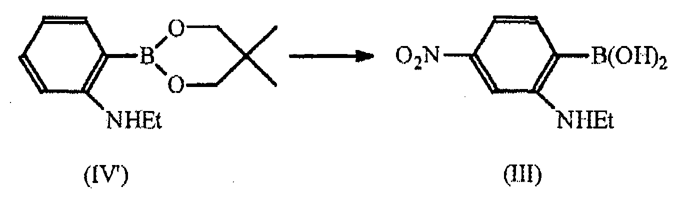 Du 2-éthylamino boronate de néopentyl glycol (0,9g, 3,9 mmoles) est dissous à froid dans 5g d'acide sulfurique à 98%. La solution est traitée par l'addition à 0-5°C d'un mélange d'acide nitrique 98% (0,36g, 1,5 éq) et d'acide sulfurique 98% (1,14g). Après 2 heures d'agitation, le milieu est versé dans 10g d'eau à 0-10°C. Le mélange obtenu est neutralisé par de l'ammoniaque à 32% (≈ 8g). Le pH est ajusté autour de 7 par ajout si nécessaire d'acide sulfurique. Le précipité est filtré, lavé à l'eau (5 ml), et séché azéotropiquement avec du MTBE. Après filtration et séchage, un solide marron est obtenu (0,7g), soit un rendement de 80%.

### EXEMPLE 4 :

### Préparation de chlorure de 3,8-dinitro-5-éthyl-6-phénylphénanthridinium (I) à partir de (II) + (III).

Un mélange de 0,31g de 2-bromo-5-nitrobenzophénone et de 0,3g d'acide 2-éthylamino-4-nitrophénylboronique est dissous dans 5 ml de 1,2-diméthoxyéthane. Une solution de carbonate de sodium (2g à 16% dans l'eau) et du tétrakis-triphénylphosphine palladium (O) (0,05g) sont ajoutés sous azote. Le mélange est porté au reflux pendant 20 heures. L'analyse HPLC de la solution obtenue donne un rendement en 3,8-dinitro-5-éthyl-6-phénylphénanthridinium de 15 à 20%.

### EXEMPLE 4 :

### Variante de l'exemple 4.

Un mélange de 2-bromo-5-nitrobenzophénone (0,31g, 1 éq) et d'acide 2-éthylamino-4-nitrophénylboronique (0,3g, 12 éq) est dissous dans 5 ml de 1,2-diméthoxyéthane. Du carbonate de césium (1g, 3 éq) et dichloro(diphénylphosphinoferracène) palladium (II) (0,05g, 0,05 éq) sont ajoutés sous azote. Le mélange est porté au reflux pendant 8 heures. Le dosage du 3,8-dinitro-5-éthyl-6-phénylphénanthridinium donne un rendement de 10 à 15%.

## Revendications

1. Procédé utile pour la préparation de sels quaternaires de phénanthridinium de formule générale (I) : 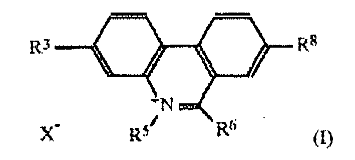 dans laquelle:
- au moins un des groupement R³ et R⁸ représente un groupement nitro (NO₂),
- R⁵ représente un groupement méthyle ou éthyle,
- R⁶ représente un groupement aryle ou hétéroaryle en C₅ à C₁₂, condensé ou non, et pouvant incorporer un ou plusieurs hétéroatomes et,
- X- représente un anion halogénure ou hydrogénosulfate ou la base conjuguée d'un acide minéral,
**caractérisé en ce qu'**il comprend la réaction d'au moins un dérivé halogénoaromatique de formule (II) : 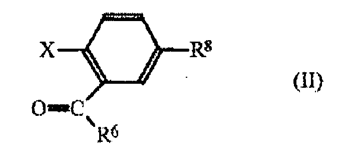 dans laquelle R⁸ et R⁶ sont tels que définis précédemment et X représente un atome d'halogène,
avec au moins un dérivé phénylboronique de formule (III) : 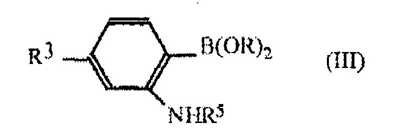 dans laquelle:
- R³ et R⁵ sont tels que définis précédemment et,
- les deux groupements R représentent un atome d'hydrogène ou figurent ensemble un radical cyclique de formule : 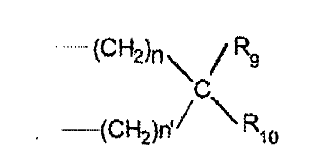
dans laquelle:
- n et n' représentent indépendamment l'un de l'autre un entier compris entre 0 et 4, et
- R₉ et R₁₀ figurent indépendamment l'un de l'autre un atome d'hydrogène ou un groupe alkyle linéaire ou ramifié en C₁ à C₅,
en milieu basique et en présence d'une qualité efficace d'un catalyseur de palladium et, **en ce que** l'on récupère ledit produit de formule générale (1).

2. Procédé selon l'une quelconque des revendications précédentes, **caractérisé en ce que** R⁶ représente un groupement phényle.

3. Procédé selon l'une quelconque des revendications précédentes, **caractérisé en ce que** l'on met en présence au moins un équivalent du composé de formule (II) pour 0,8 à 1,5 de composé de formule (III).

4. Procédé selon l'une quelconque des revendications précédentes, **caractérisé en ce que** le catalyseur est à base de palladium (o).

5. Procédé selon la revendication 4, **caractérisé en ce que** le catalyseur à hase de palladium (o) est Pd(PPh₃)₄.

6. Procédé selon l'une quelconque des revendications précédentes, **caractérisé en ce que** la base est choisie parmi les hydroxydes alcalins, les carbonates et hydrogénocarbonates, les alcoolates, les phosphates et les bases azotées aliphatiques ou cycloniques.

7. Procédé selon la revendication 6, **caractérisé en que** la base est CS₂CO₃, Et₃N ou Bu₃N.

8. Procédé selon l'une quelconque des revendications précédentes, **caractérisé en ce que** la réaction est réalisée à une température supérieure à 60°C.

9. Procédé selon l'une quelconque des revendications précédentes, **caractérisé en ce qu'**il comprend:
- le mélange des composés de formules générales (II) et (III) dans un solvant,
- l'ajout au dit mélange de la base et du catalyseur,
- le chauffage de l'ensemble du milieu réactionnel à une température supérieure à 60°C, et
- la récupération du composé de formule générale (I), par acidification du dit milieu réactionnel.

10. Procédé selon l'une des revendications précédentes, utile pour la préparation du sel de 3,8-dinitro-5-éthyl-6-phénylphénanthridinium.

11. Procédé selon l'une quelconque des revendications précédentes, **caractérisé en ce que** le dérivé phénylboronique de formule (III) est préparé à partir d'un composé de formule générale (VI) : 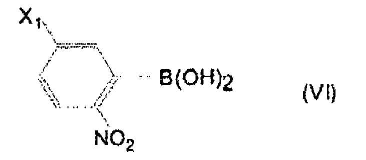 dans laquelle X₁ représente un atome d'halogène,
ledit composé de formule générale (VI) étant préparé par nitration d'un composé de formule générale (VII) : 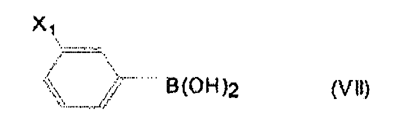 avec X₁ tel que défini précédemment,
ladite nitration étant réalisée dans de l'anhydride acétique ou un solvant analogue.

12. Procédé selon la revendication 11, **caractérisé en ce que** le composé de formule générale (VI) est obtenu avec une régiosélectivité au moins supérieure à 95%

13. Procédé selon la revendication 11 ou la revendication 12, **caractérisé en ce que** la préparation du dérivé phénylboronique de formule (III) à partir du composé de formule générale (VI) comprend :
- une hydrogénation du composé de formule (VI) avec H₂ permettant d'effectuer simultanément la réduction de la fonction nitro en fonction amino et une déhalogénation pour obtenir un composé de formule générale (V) : 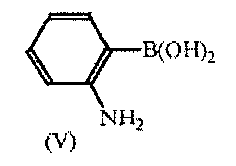
- une monoalkylation au niveau de l'azote du composé de formule (V) pour donner l'acide 2-alkylphénylboronique de formule générale (IV) : 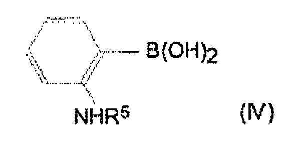 dans laquelle, R⁵ est tel que défini précédemment, et
- une nitration du composé de formule (IV) par le mélange acide nitrique/acide sulfurique à 0-5°C pour obtenir le dérivé phénylboronique de formule (III).

14. Procédé selon la revendication 11 ou la revendication 12, **caractérisé en ce que** la préparation du dérivé phénylboronique de formule (III) à partir du composé de formule générale (VI) comprend :
- une hydrogénation du composé de formule (VI) avec H₂ permettant d'effectuer simultanément la réduction de la fonction nitro en fonction amino et une déhalogénation pour obtenir un composé de formule générale (V) : 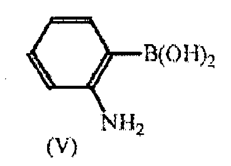
- une réaction du composé de formule (V) avec le 2,2-diméthyl-1,3-propane diol dans le THF pour donner le 5,5-diméthyl-2(2-aminophényl)-1,3,2 dioxaborinane de formule (V') : 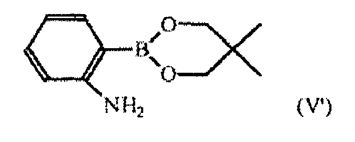
- une monoalkylation au niveau de l'azote du composé de formule (V') pour donner le 5,5-diméthyl-2(2-alkylaminophényl)-1,3,2-dioxaborinane, et
- une nitration du 5,5-dimiéthyl-2(2-alkylaminophényl)-1,3,2-dioxaborinane par le mélange acide nitrique/acide sulfurique à 0-5°C pour obtenir le dérivé phénylboronique de formule (III).

15. Composé de formule générale (VI) **caractérisé en ce que** ledit composé est préparé par un procédé selon l'une quelconque des revendications 11 à 14.

16. Composé selon la revendication 15, **caractérisé en ce qu'**il s'agit de l'acide 5-chloro-2-nitrophénylboronique.

## Claims

1. Process for the preparation of quaternary salts of phenanthridinium having the general formula (I): 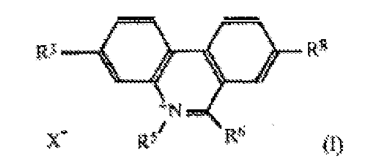 in which:
- at least one of the groups R³ and R⁸ is a nitro group (NO₂),
- R⁵ is a methyl or ethyl group,
- R⁶ is an aryl group or heteroaryl group C₅ to C₁₂, which may or may not be condensed, and may incorporate one or more hetero atoms and,
- X- is a halide or hydrogenosulphate anion or the conjugate base of a mineral acid,
**characterised in that** it comprises reacting at least one haloaromatic derivative of formula (II): 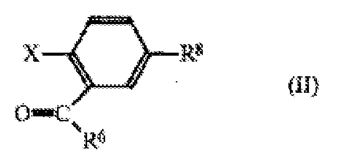 in which R⁸ and R⁶ are such as defined previously and X is a halogen atom, with at least one phenylboronic derivative of formula (III): 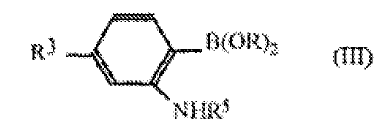 in which:
- R³ and R⁵ are such as defined previously and,
- the two R groups represent a hydrogen atom or together denote a cyclic radical of the formula: 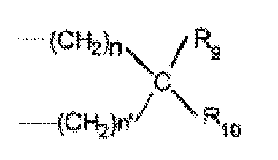
in which:
- n and n' represent independently of one another an integer between 0 and 4, and
- R₉ and R₁₀ denote independently of one another a hydrogen atom or a C₁ - C₅ linear or branched alkyl group,
in a basic medium and in the presence of an effective quantity of a palladium catalyst, and **in that** said product of general formula (I) is recovered.

2. Process according to any one of the preceding claims, **characterised in that** R⁶ is a phenyl group.

3. Process according to any one of the preceding claims, **characterised in that** at least one equivalent of the compound of formula (II) for 0.8 to 1.5 of the compound of formula (III) are brought together.

4. Process according to any one of the preceding claims, **characterised in that** the catalyst is based on palladium (o).

5. Process according to claim 4, **characterised in that** the catalyst based on palladium (o) is Pd(PPh₃)₄.

6. Process according to any one of the preceding claims, **characterised in that** the base is selected from alkaline hydroxydes, carbonates and hydrogenocarbonates, alcoholates, phosphates and aliphatic or cyclanic nitrogen bases.

7. Process according to claim 6, **characterised in that** the base is CS₂CO₃, Et₃N or Bu₃N.

8. Process according to any one of the preceding claims, **characterised in that** the reaction is conducted at a temperature above 60°C.

9. Process according to any one of the preceding claims, **characterised in that** it comprises:
- mixing of the compounds of general formulae (II) and (III) in a solvent,
- addition to said mixture of the base and the catalyst,
- heating of the whole of the reaction medium to a temperature above 60°C, and
- recovery of the compound of general formula (I), by acidification of the said reaction medium.

10. Process according to any of the preceding claims, useful for the preparation of the salt of 3,8-dinitro-5-ethyl-6-phenylphenanthridinium.

11. Process according to any one of the preceding claims, **characterised in that** the phenylboronic derivative of formula (III) is prepared from a compound of the general formula (VI): 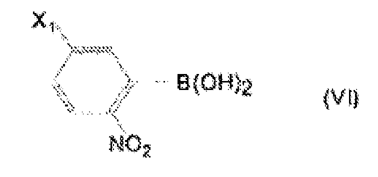 in which X₁ is a halogen atom,
the said compound of general formula (VI) being prepared by nitration of a compound of the general formula (VII): 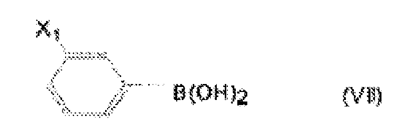 with X₁ such as defined previously,
the said nitration being conducted in acetic anhydride or an analogous solvent.

12. Process according to claim 11, **characterised in that** the compound of general formula (VI) is obtained with a regioselectivity at least greater than 95%.

13. Process according to claim 11 or claim 12, **characterised in that** the preparation of the phenylboronic derivative of formula (III) from the compound of general formula (VI) comprises:
- hydrogenation of the compound of formula (VI) with H₂ enabling simultaneous reduction of the nitro group to amine and dehalogenation to obtain a compound of general formula (V): 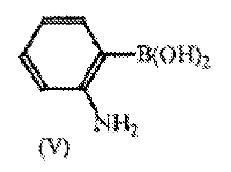
- monoalkylation at the nitrogen level of the compound of formula (V) to give 2-alkylphenylboronic acid of general formula (IV): 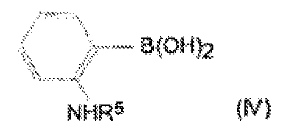 in which R⁵ is such as defined previously, and
- nitration of the compound of formula (IV) by the nitric acid/sulphuric acid mixture at 0-5°C to obtain the phenylboronic derivative of formula (III).

14. Process according to claim 11 or claim 12, **characterised in that** the preparation of the phenylboronic derivative of formula (III) from the compound of general formula (VI) comprises:
- hydrogenation of the compound of formula (VI) with H₂ enabling simultaneous reduction of the nitro group to amine and dehalogenation to obtain a compound of general formula (V): 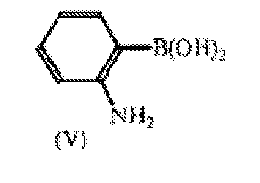
- reaction of the compound of formula (V) with 2,2-dimethyl-1,3-propane diol in THF to give 5,5-dimethyl-2(2-aminophenyl)-1,3,2 dioxaborinane of formula (V'): 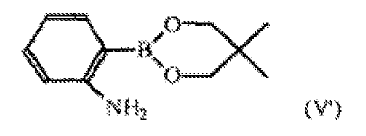
- monoalkylation at the nitrogen level of the compound of formula (V') to give 5,5-dimethyl-2(2-alkylaminophenyl)-1,3,2-dioxaborinane, and
- nitration of the 5,5-dimethyl-2(2-alkylaminophenyl)-1,3,2-dioxaborinane by the nitric acid/sulphuric acid mixture at 0-5°C to obtain the phenylboronic derivative of formula (III).

15. Compound of the general formula (VI) **characterised in that** the said compound is prepared by a process according to any one of claims 11 to 14.

16. Compound according to claim 15, **characterised in that** it is 5-chloro-2-nitrophenylboronic acid.

## Patentansprüche

1. Verfahren zur Herstellung von quaternären Phenanthridiniumsalzen der allgemeinen Formel (I): 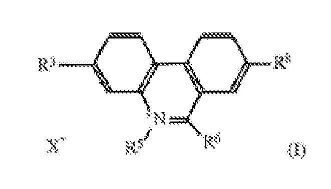 wobei:
- mindestens einer der Reste R³ und R⁸ eine Nitrogruppe (NO₂) darstellt,
- R⁵ eine Methyl- oder Ethylgruppe darstellt,
- R⁶ einen Arylrest oder Heteroarylrest von C₅-C₁₂, darstellt, kondensiert oder nicht kondensiert, und ein oder mehrere Heteroatome einschließen kann und
- X- ein Halogenid- oder Hydrogensulfat-Anion oder die konjugierte Base einer Mineralsäure darstellt,
**dadurch gekennzeichnet, dass** das Verfahren die Umsetzung mindestens eines halogenaromatischen Derivats der Formel (II): 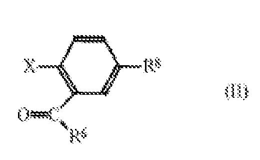 wobei R⁸ und R⁶ wie vorstehend definiert sind und X ein Halogenatom darstellt, mit mindestens einem Phenylborderivat der Formel (III) umfasst: 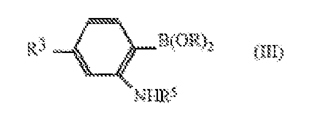 wobei:
- R³ und R⁵ wie vorstehend definiert sind und
- die beiden Reste R ein Wasserstoffatom darstellen oder zusammen einen cyclischen Rest der Formel 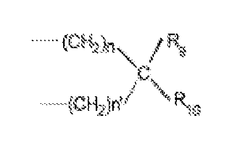 darstellen,
wobei:
- n und n' unabhängig voneinander eine ganze Zahl von 0 bis 4 darstellen und
- R₉ und R₁₀ unabhängig voneinander ein Wasserstoffatom oder einen linearen oder verzweigten C₁-C₅-Alkylrest darstellen,
in basischem Medium und in Gegenwart einer wirksamen Menge eines Palladiumkatalysators, und dass das Produkt der allgemeinen Formel (I) gewonnen wird.

2. Verfahren gemäß einem der vorhergehenden Ansprüche, **dadurch gekennzeichnet, dass** R⁶ eine Phenylgruppe darstellt.

3. Verfahren gemäß einem der vorhergehenden Ansprüche, **dadurch gekennzeichnet, dass** man mindestens ein Äquivalent der Verbindung der Formel (II) mit 0,8 bis 1,5 der Verbindung von Formel (III) zusammen bringt.

4. Verfahren gemäß einem der vorhergehenden Ansprüche, **dadurch gekennzeichnet, dass** der Katalysator auf Palladium (O) basiert.

5. Verfahren gemäß Anspruch 4, **dadurch gekennzeichnet, dass** der Katalysator auf Basis von Palladium (O) Pd(PPh₃)₄ ist.

6. Verfahren gemäß einem der vorhergehenden Ansprüche, **dadurch gekennzeichnet, dass** die Base aus Alkalihydroxiden, Carbonaten und Hydrogencarbonaten, Alkoholaten, Phosphaten und aliphatischen oder cycloalkanischen Stickstoffbasen ausgewählt ist.

7. Verfahren gemäß Anspruch 6, **dadurch gekennzeichnet, dass** die Base CS₂CO₃, Et₃N oder Bu₃N ist.

8. Verfahren gemäß einem der vorhergehenden Ansprüche, **dadurch gekennzeichnet, dass** die Umsetzung bei einer Temperatur von über 60°C durchgeführt wird.

9. Verfahren gemäß einem der vorhergehenden Ansprüche, **dadurch gekennzeichnet, dass** es umfasst:
- das Mischen der Verbindungen der allgemeinen Formeln (II) und (III) in einem Lösungsmittel,
- das Zugeben der Base und des Katalysators zu dem Gemisch,
- das Erwärmen des gesamten Reaktionsmediums auf eine Temperatur von über 60°C und
- das Gewinnen der Verbindung der allgemeinen Formel (I) durch Ansäuern des Reaktionsmediums.

10. Verfahren gemäß einem der vorhergehenden Ansprüche zur Herstellung von 3,8-Dinitro-5-ethyl-6-phenylphenanthridiniumsalz.

11. Verfahren gemäß einem der vorhergehenden Ansprüche, **dadurch gekennzeichnet, dass** das Phenylborderivat der Formel (III) ausgehend von einer Verbindung der allgemeinen Formel (VI) hergestellt wird: 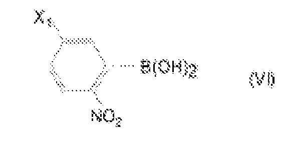 wobei X₁ ein Halogenatom darstellt,
wobei die Verbindung der allgemeinen Formel (VI) durch Nitrierung einer Verbindung der allgemeinen Formel (VII) hergestellt wird: 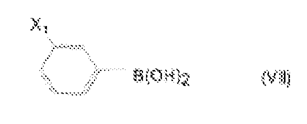 in der X₁ wie vorstehend definiert ist,
wobei die Nitrierung in Essigsäureanhydrid oder einem analogen Lösungsmittel durchgeführt wird.

12. Verfahren gemäß Anspruch 11, **dadurch gekennzeichnet, dass** die Verbindung der allgemeinen Formel (VI) mit einer Regioselektivität von mindestens mehr als 95% erhalten wird.

13. Verfahren gemäß Anspruch 11 oder Anspruch 12, **dadurch gekennzeichnet, dass** die Herstellung des Phenylborderivats der Formel (III) aus der Verbindung der allgemeinen Formel (VI) umfasst:
- eine Hydrierung der Verbindung der Formel (VI) mit H₂, was es erlaubt, gleichzeitig die Nitrofunktion in der Aminofunktion zu reduzieren und eine Dehalogenierung durchzuführen, um eine Verbindung der allgemeinen Formel (V) zu erhalten: 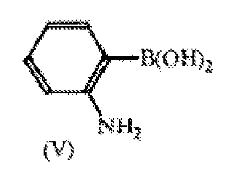
- eine Monoalkylierung am Stickstoff der Verbindung der Formel (V), um eine 2-Alkylphenylborsäure der allgemeinen Formel (IV) zu ergeben: 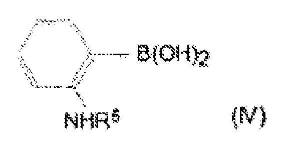 wobei R⁵ wie vorstehend definiert ist und
- eine Nitrierung der Verbindung der Formel (IV) durch eine Mischung aus Salpetersäure/Schwefelsäure bei 0-5°C, um das Phenylborderivat der Formel (III) zu erhalten.

14. Verfahren gemäß Anspruch 11 oder Anspruch 12, **dadurch gekennzeichnet, dass** die Herstellung des Phenylborderivats der Formel (III) aus der Verbindung der allgemeinen Formel (VI) umfasst:
- eine Hydrierung der Verbindung der Formel (VI) mit H₂, was es erlaubt, gleichzeitig die Nitrofunktion in der Aminofunktion zu reduzieren und eine Dehalogenierung durchzuführen, um eine Verbindung der allgemeinen Formel (V) zu erhalten: 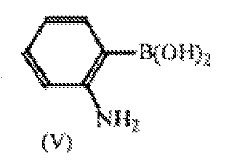
- eine Umsetzung der Verbindung der Formel (V) mit 2,2-Dimethyl-1,3-propandiol in THF, um 5,5-Dimethyl-2-(2-aminophenyl)-1,3,2-dioxaborinan der Formel (V') zu ergeben: 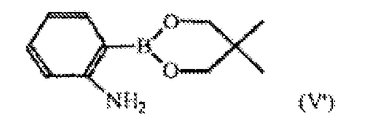
- eine Monoalkylierung am Stickstoff der Verbindung der Formel (V'), um 5,5-Dimethyl-2-(2-alkylaminophenyl)-1,3,2-dioxaborinan zu ergeben und
- eine Nitrierung von 5,5-Dimethyl-2-(2-alkylaminophenyl)-1,3,2-dioxaborinan durch eine Mischung aus Salpetersäure/Schwefelsäure bei 0-5°C, um das Phenylborderivat der Formel (III) zu erhalten.

15. Verbindung der allgemeinen Formel (VI), **dadurch gekennzeichnet, dass** die Verbindung durch ein Verfahren gemäß einem der Ansprüche 11 bis 14 hergestellt wird.

16. Verbindung gemäß Anspruch 15, **dadurch gekennzeichnet, dass** es sich um 5-Chlor-2-nitrophenylborsäure handelt.
